# EUROPÄISCHE PATENTSCHRIFT

(11) **EP 1 989 160 B1**
(45) Veröffentlichungstag und Bekanntmachung des Hinweises auf die Patenterteilung: **09.04.2014**
(21) Anmeldenummer: 07704544.1
(22) Anmeldetag: 13.02.2007
(51) Int. Cl.: C07C 2/78, C07C 2/80, C07C 11/24

(54) **VERFAHREN ZUR HERSTELLUNG VON ACETYLEN**
METHOD FOR PRODUCING ACETYLENE
PROCÉDÉ DE PRODUCTION D'ACÉTYLÈNE

(30) Priorität: 21.02.2006 US 775158 P
(43) Veröffentlichungstag der Anmeldung: 12.11.2008
(73) Patentinhaber: BASF SE, 67056 Ludwigshafen (DE)
(72) Erfinder: BACHTLER, Michael, 76857 Albersweiler (DE); EHRHARDT, Kai Rainer, 67346 Speyer (DE); WITTE, Christopher P, Prairieville, La 70769 (US); HAYES, Michael L., Gonzales, Louisiana 70737 (US)
(86) Internationale Anmeldenummer: PCT/EP2007/051370
(87) Internationale Veröffentlichungsnummer: WO 2007/096271

(56) Entgegenhaltungen:
- US-A- 2 822 411
- US-A- 4 767 569
- US-A- 5 824 834
- MATTHIAS BOHNET ET AL.: "ULLMANN'S ENCYCLOPEDIA OF INDUSTRIAL CHEMISTRY SIXTH COM. REV. ED. VOL. 1" 2003, WILEY-VCH VERLAG GMBH & CO. KGAA WEINHEIM , XP002442775 in der Anmeldung erwähnt Seiten 225-231

## Beschreibung

Die vorliegende Erfindung betrifft ein verbessertes Verfahren zur Herstellung von Acetylen aus Kohlenwasserstoffen durch partielle Oxidation, Spaltung im Lichtbogen oder Pyrolyse, wobei man den das gewonnene Acetylen und Ruß enthaltenden Stoffstrom einem oder mehreren Schraubenkompressoren zuführt.

Acetylen wird industriell unter anderem nach dem von der BASF entwickelten Verfahren hergestellt, das auf partieller Oxidation von Kohlenwasserstoffen (bevorzugt Erdgas) mit Sauerstoff beruht. Es ist beispielsweise in der US 5,824,834 und in "Ullmann's Encyclopedia of Industrial Chemistry, Sixth Edition, 2000, Electronic Release, Chapter 4.2.1" beschrieben.

Die beiden Einsatzstoffe Kohlenwasserstoff und Sauerstoff werden zuerst vorgewärmt, bei Einsatz von Erdgas auf ca. 500 bis 650°C, dann werden sie gemischt und erst danach im Feuerraum in einer Flamme umgesetzt, die durch den sogenannten Brennerblock stabilisiert wird. Vorwärmung und -mischung sind für hohe Acetylenausbeuten erforderlich.

Die Flammenreaktion bei Temperaturen über etwa 1500°C wird durch Eindüsen von Wasser nach wenigen Millisekunden gequencht, d.h. die sehr schnelle Abkühlung auf ca. 90°C bricht die Radikal-Kettenreaktion in der Flamme ab. Hierdurch wird der Abbau des thermodynamisch instabilen Zwischenprodukts Acetylen verhindert. Das Reaktionsprodukt ist das sogenannte Spaltgas, das ein Gemisch aus Acetylen, Roh-Synthesegas (hauptsächlich H₂ und CO), Wasserdampf und Nebenprodukten ist. Eines der Nebenprodukte hierbei ist Ruß. Vor der Kompression wird das Spaltgas üblicherweise auf circa 30-50°C gekühlt, um den Großteils des Wasserdampfes zu kondensieren und dadurch die zu komprimierende Gasmenge zu reduzieren.

Der als Nebenprodukt gebildete Ruß erweist sich in den nachfolgenden Prozessstufen als störend und zur Steigerung der Effektivität des Verfahrens ist man daher bemüht, ihn von dem Spaltgas abzutrennen.

Der Ruß entsteht in der Flamme während der partiellen Oxidation der Kohlenwasserstoffe. Da der Ruß in der Gasphase gebildet wird handelt es sich um sehr feine Partikel, die üblicherweise kleiner als 1 µm sind. Die gebildete Rußmenge ist abhängig von der Fahrweise des Reaktors, wie es in US-5824834 beschrieben ist. Der Großteil des Rußes wird durch das Wasser im Brennerquench und in der nachgeschalteten Kühlkolonne abgeschieden. Die dann jedoch noch in dem zu komprimierenden Spaltgas enthaltene Rußmenge ist noch beträchtlich, sie liegt beim BASF-Verfahren üblicherweise in einer Konzentration von etwa 100 bis 1000 mg/Nm³ vor, sie kann jedoch bei anderen Verfahren auch deutlich höher liegen. Um Schäden an den für die Kompression des Gases eingesetzten Verdichter zu vermeiden, wird der Ruß nach dem Stand der Technik vor der Kompression des Spaltgases abgeschieden, wobei der Rußgehalt im gereinigten Spaltgas unterhalb etwa 20 mg/Nm³ liegt. Für die Abscheidung können z.B. Nass-Elektrofilter eingesetzt werden.

Nach der Rußabtrennung und der Kühlung des Spaltgases werden üblicherweise Schraubenkompressoren zur Verdichtung des Spaltgases eingesetzt, wie sie z.B. von den Firmen GE Oil and Gas, Inc., 3300 Medaust Drive, Oshkosh, WI 54902, USA, MAN Turbomaschinen AG, 46145 Oberhausen, Germany oder Kobe Steel, LTD., Compressor division, 9-12, Kita-shinagawa 5-chrome, Shinagawa-ku, Tokyo, 141-8688, Japan gefertigt werden. Diese teuren und hochpräzise gefertigten Maschinen werden bevorzugt eingesetzt, da sie in der Lage sind, auch Gase zu komprimieren, welche eine Neigung zur Bildung von Polymerablagerungen aufweisen. Dem Verdichter wird dabei während des Betriebs eine geringe Menge Wasser zur Kühlung beigegeben, dieses Wasser verdampft während der Verdichtung (also in der Maschine) jedoch üblicherweise vollständig und die Schraubenkompressoren werden somit "trocken" betrieben.

Die Abscheidung des Rußes ist aufgrund seiner extremen Feinheit eine sehr anspruchsvolle Trennaufgabe, die folglich große Investitionen und hohen verfahrenstechnischen Aufwand bedingen. Ferner sind die üblicherweise zum Einsatz kommenden Nass-Elektrofilter gerade für die Abscheidung des Acetylenrußes immer wieder die Ursache von Betriebsunterbrechungen. Häufige Ursachen sind beispielsweise gerissene Sprühdrähte, wobei die im Spaltgas enthaltenen organischen Säuren hierfür mitverantwortlich sind, und die Bildung von Ablagerungen, die durch die schlechte Benetzung des Rußes durch Wasser gefördert werden.

Es stellte sich somit die Aufgabe, ein verbessertes Verfahren zur Herstellung von Acetylen zu finden, welches die genannten Nachteile vermeidet und das eine Herstellung des Acetylens in effektiver und verfahrenstechnisch einfacher Art und Weise mit hoher Verfügbarkeit und bei hohen Standzeiten ermöglicht.

Demgemäß wurde ein Verfahren zur Herstellung von Acetylen aus Kohlenwasserstoffen durch partielle Oxidation, Spaltung im Lichtbogen oder Pyrolyse gefunden, wobei man den das gewonnene Acetylen und Ruß enthaltenden Stoffstrom einem oder mehreren Schraubenkompressoren, die ein- oder mehrstufig sind zuführt, welches dadurch gekennzeichnet ist, dass man in den Schraubenkompressor eine Flüssigkeit einspritzt, welche den größten Teil des in dem Stoffstrom enthaltenen Rußes aufnimmt.

Es wurde überraschend festgestellt, dass die als Verdichter eingesetzten Schraubenkompressoren in der Lage sind, beachtliche Mengen an Ruß aus dem Spaltgas abzuscheiden, falls soviel Wasser am Eintritt eingedüst wird, dass Wasser in flüssiger Form aus dem Kompressor austritt, in dem der abgeschiedene Ruß dispergiert ist. Folglich bestimmt erfindungsgemäß die Feststoffabscheidung und nicht die Gaskühlung die eingespritzte Wassermenge. Dadurch kann vorteilhafterweise auf einen Apparat zur Abscheidung des Rußes (üblicherweise der Elektrofilter) verzichtet werden, und der Spaltgasstrom kann somit erfindungsgemäß direkt auf den Schraubenkompressor geführt werden. Zwischen Reaktor und Kompressor können weitere Verfahrensschritte geschaltet sein, falls dies die Anwesenheit von Ruß zulässt. Bevorzugt wird der Spaltgasstrom vor der Kompression und gleichzeitigen Rußabscheidung in einer zusätzlichen Kolonne gekühlt, um den zu komprimierenden Volumenstrom durch Kondensation von Wasserdampf zu verringern und um die Temperatur am Kompressoraustritt zu senken. Die Eliminierung der Rußabscheider (üblicherweise Elektrofilter) vereinfacht bei einer Neuanlage deutlich den Aufwand und senkt signifikant die Investitionskosten. Weiterhin wird für alle Anlagen die Verfügbarkeit erhöht, weil die störanfälligen Elektrofilter nicht mehr betrieben werden müssen, und die Effektivität des gesamten Prozesses wird gesteigert.

Eine solche Verfahrensführung hätte der Fachmann nicht in Erwägung gezogen, handelt es sich doch bei Schraubenkompressoren um sehr teure und hochpräzise gefertigte Apparate. So ist beispielsweise bei den für die hier vorherrschenden Gasströme einerseits eine große Dimensionierung der Schrauben erforderlich, andererseits muss trotz dieser beachtlichen Abmessungen ein konstant minimaler Abstand zwischen den sich in sehr hoher Geschwindigkeit drehenden Schrauben gewährleistet werden, welcher in der Größenordnung einiger weniger 1/100 mm liegt. Bei dem Betrieb dieser aufwendigen Maschinen mit hohem Feststoffgehalt, wie es der Spaltgasstrom nach Verlassen der Kühlkolonne ja noch aufweist, stand eine Beschädigung dieses Apparats zu befürchten. Überraschenderweise bewirkt jedoch die im Überschuss eingespritzte Flüssigkeit, bevorzugt Wasser, dass ein dauerhafter Betrieb möglich ist.

Bei dem erfindungsgemäßen Verfahren kann als Flüssigkeit bevorzugt ein Lösungsmittel eingespritzt werden. Das Lösungsmittel kann nach der Kompression des Stoffstroms für dessen Auftrennung (gegebenenfalls neben weiteren Lösungsmitteln) verwendet werden. Als Verdichter werden ein oder mehrere Schraubenkompressoren eingesetzt, die ein- oder mehrstufig sind. Man kann Wasser, Öl oder Lösungsmittel als rußfreie Flüssigkeit als Sperrflüssigkeit zur Abdichtung gegen die Umgebung in dem Schraubenkompressor einsetzen. Man kann Rußablagerungen auf dem dem Schraubenkompressor vorgeschalteten Saugsieb und / oder dem dem Schraubenkompressor vorgeschalteten Saugschalldämpfer durch die kontinuierliche oder diskontinuierliche Befeuchtung mit Wasser, Öl oder Lösungsmittel verhindern. Hierbei kann man diese zur Befeuchtung eingesetzte Flüssigkeit kontinuierlich mit einer Vollkegeldüse auf das Saugsieb düsen.

Die erfindungsgemäße Abscheidung des Rußes gelingt besonders gut und langfristig störungsfrei, wenn der Rußgehalt im Wasser, welches den Schraubenkompressor in flüssiger Form (ohne Wasserdampf) verlässt, zwischen 0,05 und 5 Gew-%, bevorzugt zwischen 0,1 und 1,5 Gew-% und besonders bevorzugt zwischen 0,15 und 0,8 Gew-% liegt. Höhere Rußgehalte sind zu vermeiden, weil aufgrund der extrem geringen Größe des Rußes ansonsten die Viskosität der Ruß-Wasser-Suspension zu stark ansteigt. Geringere Rußgehalte würde die Zugabe von Wasser oder geringere Kühlung des wasserdampfgesättigten Spaltgases bedingen. Diese Bedingung definiert zum einen die minimal am Kompressoreintritt einzuspritzende Wassermenge und zum anderen den maximalen Rußgehalt im eingespritzten Wasser. Ursache für die Abscheidung ist die sehr intensive Vermischung und Interaktion (letztlich Relativgeschwindigkeit) zwischen den im Spaltgas enthaltenen, fein verteilten Rußpartikeln einerseits und dem Wasser andererseits, die auch bei anderen Abscheidern wie Venturiwaschern maßgeblich ist.

In einer bevorzugten Ausführungsform existiert ein Wasserkreislauf, um den Wasserverbrauch zu verringern. Durch den Kreislauf enthält das Prozesswasser Ruß. Es kann z.B. auch in einer dem Kompressor nachgeschalteten Kühlkolonne verwendet werden. Um den Rußgehalt im Wasserkreis gemäß oben stehender Bedingung zu begrenzen, muss der aus dem Spaltgas abgeschiedene Ruß ausgeschleust werden. Dies kann beispielsweise in der Form einer Ruß-Wasser-Suspension (Prozesswasser) erfolgen, welche aus dem Prozess ausgeschleust wird.

Um eine hinreichende Abscheidung zu gewährleisten muss das Verdichtungsverhältnis mindestens 1:2 betragen und bevorzugt mindestens 1:3 betragen.

In einer weiteren Ausführungsform des erfindungsgemäßen Verfahrens kann der Schraubenverdichter auch mit einer Öleinspritzung anstelle einer Wassereinspritzung betrieben werden. Auch hier kann die erfindungsgemäße Rußabscheidung bewirkt werden. Im Gegensatz zum Wasser kann das Öl maximal etwa 20 Gew-% Ruß aufnehmen. Bei Öleinspritzung wird der Kompressor üblicherweise mit großem Ölüberschuss betrieben, so dass die Verdampfung kein Problem darstellt.

Analog sind weitere Flüssigkeiten neben Öl und Wasser erfindungsgemäß geeignet wie z.B. N-Methylpyrrolidon, Methanol, Aceton, Benzin, Benzol oder Dimethylformamid. Bevorzugt werden solche Stoffe eingesetzt, die bei den verschiedenen Acetylenverfahren bereits eingesetzt werden, beim BASF Verfahren also N-Methylpyrrolidon. Der maximale Rußgehalt in der aus dem Kompressor austretenden Flüssigkeit hängt von den jeweiligen Stoffeigenschaften ab. Letztlich muss die Viskosität der jeweiligen Suspension mit den oben genannten Wasser-Ruß-Suspensionen vergleichbar sein.

Der Ruß wird während der Kompression des Spaltgases bevorzugt praktisch vollständig abgeschieden. Es können bei dem erfindungsgemäßen Verfahren in dem den Verdichter verlassenden Gasstrom Restrußgehalte von weniger als etwa 20 mg/Nm³ erreicht werden, je nach Ausgestaltung des erfindungsgemäßen Verfahrens können auch deutlich niedrigere Restrußgehalte erreicht werden. Gegenüber dem Einsatz von Elektrofiltern ist hinsichtlich der Abscheidegrade des Rußes kein Unterschied erkennbar. Gegebenenfalls im Spaltgas verbleibende Rußspuren werden vom Lösungsmittel (N-Methylpyrrolidon), das für die nachfolgende Auftrennung des Spaltgases eingesetzt wird, aufgenommen. Da das Lösungsmittel wegen Polymerbildung kontinuierlich oder zumindest regelmäßig regeneriert werden muss, stören die Rußspuren nicht.

Für einen langfristig störungsfreien Betrieb empfiehlt es sich besonders in einer bevorzugten Ausführungsform des Verfahrens, den dem Schraubenkompressor vorgeschalteten Saugschalldämpfer und das Saugsieb stets feucht zu halten, um Rußablagerungen zu verhindern. Für die konventionelle Abreinigung von Rußablagerungen muss die Acetylenproduktion bei Einstranganlagen unterbrochen und bei Mehrstranganlagen reduziert werden.

Das erfindungsgemäße Verfahren ist anwendbar sowohl für geschlossenen Wasserquench nach US 5824834 als auch offenen Wasserquench, wie im Ullman beschrieben. Auf die anderen bekannten Verfahren zur Herstellung von Acetylen, wie sie z.B. im Ullman beschrieben sind, ist das Verfahren ebenso übertragbar.

Das erfindungsgemäße Verfahren ermöglicht eine wirtschaftliche Herstellung von Acetylen. Die Abtrennung des hierbei als Nebenprodukt anfallenden Rußes erfolgt in einer verfahrenstechnisch einfachen und mit geringem Aufwand verbundenen Art und Weise, wobei gleichzeitig hohe Standzeiten bei der Produktion erreicht werden können. Bei dem bevorzugten Einsatz von Wasser als zugegebene Flüssigkeit erweist es sich weiterhin als Vorteil, dass keine weiteren, systemfremden Komponenten dem Prozess zugeführt werden.

### Beispiel

Im Ullman findet sich eine Beschreibung des BASF Verfahrens mit Wasserquench. Gemäß US 05824834 wurde die Fahrweise der Anlage so verändert, dass die Rückführung des Prozesswassers in einem geschlossenen System ermöglicht wurde und so der Kontakt des mit Schadstoffen belasteten Prozesswassers mit der Atmosphäre verhindert werden konnte.

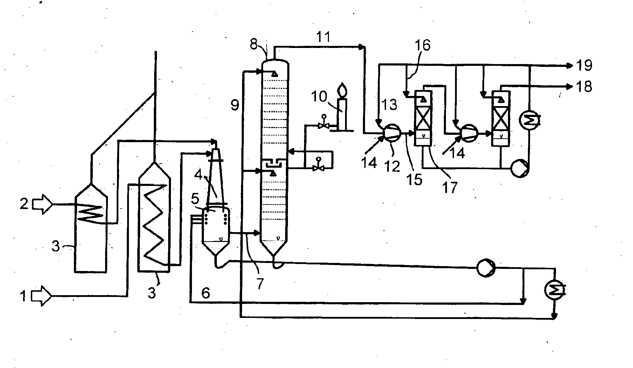

In Figur 1 ist eine Ausführungsform des erfindungsgemäßen Verfahrens dargestellt. Die Einsatzstoffe Erdgas (1) und Sauerstoff (2) werden in befeuerten Vorheizern (3) vorgewärmt. In der Mischzone (4) werden sie gemischt und im Feuerraum (5) in einer Flammenreaktion umgesetzt. Die Flamme wird unterhalb des Feuerraums durch die Eindüsung von Prozesswasser (6) gequencht. Das sogenannte Spaltgas (7), hierbei handelt es sich um den Stoffstrom, der das Acetylen und den Ruß enthält, tritt in etwa mit Kühlgrenztemperatur und mit Wasserdampf gesättigt in die Kühlkolonne (8) ein. Dort wird mit Hilfe von gekühltem Prozesswasser (9) das Spaltgas gekühlt und dadurch ein Großteil des Wasserdampfes kondensiert. Die Fackel (10) wird für An- und Abfahrvorgänge benötigt. Das auf etwa 40°C gekühlte Spaltgas (11) (45.000 Nm³/h trocken), das 200 mg/Nm³ Ruß enthält, wird nachfolgend mit Hilfe eines zweistufigen Schraubenkompressors (12) zuerst von 1,1 auf 4,2 und dann auf 11 bar (abs) komprimiert, wobei der Ruß abgeschieden wird. In jede Stufe des Kompressors werden 7,5 m³/h Prozesswasser (13) eingespritzt, das 0,15 Gew-% Ruß enthält. Für die Abdichtung zur Atmosphäre wird neben Stickstoff vollentsalztes Wasser (14) als sogenannte Sperrflüssigkeit eingesetzt, wodurch 4 m³/h in den Prozesswasserkreis eintreten. Am Austritt der ersten Stufe (15) beträgt die Temperatur 85°C und der Rußgehalt im Wasser 0,22 Gew-%. Nach jeder Kompressionsstufe wird das Spaltgas mittels gekühltem Prozesswasser (16) in Kühlkolonnen (17) auf 40°C gekühlt. Nach der Kompression wird das Spaltgas (18) in seine Bestandteile aufgetrennt, z.B. so wie im Ullman beschrieben. Das während der Kompression und nachfolgenden Kühlung kondensierte Wasser sowie das in den Kreislauf eingetragene vollentsalzte Wasser werden zusammen mit dem enthaltenen Ruß ausgeschleust (19). Der Rußgehalt im Wasserkreislauf stellt sich entsprechend der in den Kreislauf eingetragenen Wassermenge (Kondensierter Wasserdampf + vollentsalztes Wasser) und abgeschiedenen Rußmenge ein, die den als Ruß-Wasser-Suspension ausgeschleusten Mengen entsprechen.

In einem weiteren Ausführungsbeispiel blieben die Anfahrsiebe vor dem Eintritt in die beiden Kompressorstufen und nach den Saugschalldämpfern permanent installiert. Die Siebe verhindern Schäden am Kompressor durch das Eindringen von Fremdkörpern wie Schauben, die von Montage- oder Wartungstätigkeiten herstammen. Das Anfahrsieb am Eintritt der ersten Kompressorstufe wird mit 2 m³/h Wasser besprüht, um die Bildung von Rußablagerungen zu verhindern. Hierfür kommt eine handelsübliche Einstoff-Vollkegeldüse zu Einsatz, die mit einem Vordruck von 5 bar betrieben wurde. Der Öffnungswinkel und der Anstand von Anfahrsieb wurde so gewählt, dass eine möglichst gleichmäßige und vollständige Benetzung sichergestellt ist. Hierbei ist die gegebenenfalls hohe Strömungsgeschwindigkeit des Spaltgases zu berücksichtigen. Ohne Wassereindüsung musste das Anfahrsieb drei Monate gereinigt werden, wozu die Acetylenproduktion bei Einstranganlagen unterbrochen und bei Mehrstranganlagen reduziert werden muss. Das Anfahrsieb der zweiten Stufe musste in dem Ausführungsbeispiel nicht feucht gehalten werden. Folglich wird der Ruß bereits in der ersten Stufe praktisch vollständig abgeschieden.

## Patentansprüche

1. Verfahren zur Herstellung von Acetylen aus Kohlenwasserstoffen durch partielle Oxidation, Spaltung im Lichtbogen oder Pyrolyse, wobei man den das gewonnene Acetylen und Ruß enthaltenden Stoffstrom einem oder mehreren Schraubenkompressoren, die ein- oder mehrstufig sind, zuführt, **dadurch gekennzeichnet, dass** man in den Schraubenkompressor eine Flüssigkeit einspritzt, welche den größten Teil des in dem Stoffstrom enthaltenen Rußes aufnimmt.

2. Verfahren nach Anspruch 1, **dadurch gekennzeichnet, dass** man als Flüssigkeit Wasser einspritzt.

3. Verfahren nach Anspruch 1, **dadurch gekennzeichnet, dass** man als Flüssigkeit Öl einspritzt.

4. Verfahren nach Anspruch 1, **dadurch gekennzeichnet, dass** man als Flüssigkeit ein Lösungsmittel einspritzt.

5. Verfahren nach Anspruch 4, **dadurch gekennzeichnet, dass** das Lösungsmittel nach der Kompression des Stoffstroms für dessen Auftrennung gegebenenfalls neben weiteren Lösungsmitteln verwendet wird.

6. Verfahren nach Ansprüchen 1 bis 5, **dadurch gekennzeichnet, dass** das Verdichtungsverhältnis mindestens 1:2 beträgt.

7. Verfahren nach Ansprüchen 1 bis 6, **dadurch gekennzeichnet, dass** das Verdichtungsverhältnis bevorzugt mindestens 1:3 beträgt.

8. Verfahren nach Anspruch 2 oder 6 bis 7, **dadurch gekennzeichnet, dass** das aus dem Schraubenkompressor austretende Wasser einen Rußgehalt zwischen 0,05 und 5 Gew.-% besitzt.

9. Verfahren nach Anspruch 8, **dadurch gekennzeichnet, dass** das aus dem Schraubenkompressor austretende Wasser einen Rußgehalt zwischen 0,1 und 1,5 Gew-% besitzt.

10. Verfahren nach Anspruch 8, **dadurch gekennzeichnet, dass** das aus dem Schraubenkompressor austretende Wasser einen Rußgehalt zwischen 0,15 und 0,8 Gew-% besitzt.

11. Verfahren nach Ansprüchen 1 bis 10, **dadurch gekennzeichnet, dass** man rußfreie Flüssigkeit gemäß Ansprüchen 2 bis 4 als Sperrflüssigkeit zur Abdichtung gegen die Umgebung in dem Schraubenkompressor einsetzt.

12. Verfahren nach Ansprüchen 1 bis 11, **dadurch gekennzeichnet, dass** man Rußablagerungen auf dem dem Schraubenkompressor vorgeschalteten Saugsieb und/oder dem dem Schraubenkompressor vorgeschalteten Saugschalldämpfer durch die kontinuierliche oder diskontinuierliche Befeuchtung mit der Flüssigkeit gemäß Ansprüchen 2 bis 4 verhindert.

13. Verfahren nach Anspruch 12, **dadurch gekennzeichnet, dass** man die Flüssigkeit kontinuierlich mit einer Vollkegeldüse auf das Saugsieb düst.

## Claims

1. A process for the preparation of acetylene from hydrocarbons by partial oxidation, arc cleavage or pyrolysis, the material stream comprising the acetylene and soot obtained being fed to one or more one-stage or multistage screw compressors, wherein a liquid which takes up the major part of the soot present in the material stream is sprayed into the screw compressor.

2. The process according to claim 1, wherein the liquid sprayed in is water.

3. The process according to claim 1, wherein the liquid sprayed in is oil.

4. The process according to claim 1, wherein the liquid sprayed in is a solvent.

5. The process according to claim 4, wherein, after the compression of the material stream, the solvent is used for the separation thereof if appropriate in addition to further solvents.

6. The process according to claims 1 to 5, wherein the compression ratio is at least 1:2.

7. The process according to claims 1 to 6, wherein the compression ratio is preferably at least 1:3.

8. The process according to claim 2 or 6 to 7, wherein the water emerging from the screw compressor has a soot content of from 0.05 to 5% by weight.

9. The process according to claim 8, wherein the water emerging from the screw compressor has a soot content of from 0.1 to 1.5% by weight.

10. The process according to claim 8, wherein the water emerging from the screw compressor has a soot content of from 0.15 to 0.8% by weight.

11. The process according to claims 1 to 10, wherein the soot-free liquid according to claims 2 to 4 is used in the screw compressor as sealing liquid for sealing from the environment.

12. The process according to claims 1 to 11, wherein soot deposits on the intake screen upstream of the screw compressor and/or on the intake muffler upstream of the screw compressor are prevented by continuous or discontinuous moistening with the liquid according to claims 2 to 4.

13. The process according to claim 12, wherein the liquid is sprayed continuously onto the intake screen using a solid-cone nozzle.

## Revendications

1. Procédé pour la préparation d'acétylène à partir d'hydrocarbures par oxydation partielle, dissociation dans un arc électrique ou par pyrolyse, en alimentant le flux de substances, contenant l'acétylène obtenu et la suie, dans un ou plusieurs compresseurs à vis, qui sont à un ou plusieurs étages, **caractérisé en ce qu'**on injecte, dans le compresseur à vis, un liquide qui absorbe la plus grande partie de la suie contenue dans le flux de substances.

2. Procédé selon la revendication 1, **caractérisé en ce qu'**on injecte de l'eau comme liquide.

3. Procédé selon la revendication 1, **caractérisé en ce qu'**on injecte de l'huile comme liquide.

4. Procédé selon la revendication 1, **caractérisé en ce qu'**on injecte un solvant comme liquide.

5. Procédé selon la revendication 4, **caractérisé en ce que** le solvant est utilisé, le cas échéant en plus d'autres solvants, après la compression du flux de substances pour sa séparation.

6. Procédé selon les revendications 1 à 5, **caractérisé en ce que** le rapport de compression est d'au moins 1:2.

7. Procédé selon les revendications 1 à 6, **caractérisé en ce que** le rapport de compression est de préférence d'au moins 1:3.

8. Procédé selon la revendication 2 ou 6 à 7, **caractérisé en ce que** l'eau sortant du compresseur à vis présente une teneur en suie entre 0,05 et 5% en poids.

9. Procédé selon la revendication 8, **caractérisé en ce que** l'eau sortant du compresseur à vis présente une teneur en suie entre 0,1 et 1,5% en poids.

10. Procédé selon la revendication 8, **caractérisé en ce que** l'eau sortant du compresseur à vis présente une teneur en suie entre 0,15 et 0,8% en poids.

11. Procédé selon les revendications 1 à 10, **caractérisé en ce qu'**on utilise, dans le compresseur à vis, un liquide exempt de suie selon les revendications 2 à 4 comme liquide de blocage pour réaliser l'étanchéité par rapport à l'environnement.

12. Procédé selon les revendications 1 à 11, **caractérisé en ce qu'**on empêche des dépôts de suie sur le tamis d'aspiration disposé en amont du compresseur à vis et/ou sur le silencieux d'aspiration disposé en amont du compresseur à vis par l'humidification continue ou discontinue au moyen du liquide selon les revendications 2 à 4.

13. Procédé selon la revendication 12, **caractérisé en ce qu'**on injecte le liquide en continu à l'aide d'un gicleur à cône plein sur le tamis d'aspiration.
